# EUROPEAN PATENT SPECIFICATION

(11) **EP 4 151 158 B1**
(45) Date of publication and mention of the grant of the patent: **21.01.2026**
(21) Application number: 21802951.0
(22) Date of filing: 14.05.2021
(51) Int. Cl.: A61B 10/00, A61B 1/00

(54) **BIOMEDICAL TUBE AND BIOMETRIC DEVICE**
BIOMEDIZINISCHER SCHLAUCH UND BIOMETRISCHE VORRICHTUNG
TUBE BIOMÉDICAL ET DISPOSITIF BIOMÉTRIQUE

(30) Priority: 15.05.2020 JP 2020086095
(43) Date of publication of application: 22.03.2023
(73) Proprietor: Kyocera Corporation, Kyoto-shi Kyoto 612-8501 (JP); OSAKA UNIVERSITY, Suita-shi Osaka 565-0871 (JP)
(72) Inventor: KOMADA, Daisuke, Kyoto-shi, Kyoto 612-8501 (JP); OSANAI, Makoto, Suita-shi, Osaka 565-0871 (JP)
(74) Representative: Viering, Jentschura & Partner mbB Patent- und Rechtsanwälte
(86) International application number: PCT/JP2021/018508
(87) International publication number: WO 2021/230377

(56) References cited:
- WO-A1-2017/170662
- WO-A1-2017/170662
- WO-A1-2020/045669
- JP-A- 2005 533 533
- JP-A- 2012 239 669
- JP-A- 2013 500 109
- US-A1- 2012 281 218
- US-A1- 2018 228 374
- US-A1- 2018 228 375
- IKEDA, NAOTO; LU, WENGUANG; MATSUNAGA, TADAO; TSURUOKA, NORIKO: "31pm2-PS-154 Thin tube-shaped neural electrodes with endoscopic observation function", PROCEEDINGS OF THE 35TH SENSOR MICROMACHINES AND APPLIED SYSTEMS SYMPOSIUM, vol. 35, 23 October 2018 (2018-10-23), Japan, pages 1 - 4, XP009541018
- IKEDA, NAOTO; LU, WENGUANG; MATSUNAGA, TADAO; TSURUOKA, NORIKO; MUSHIAKE, HAJIME; OSANAI, MAKOTO; OSHIRO, TOMOKAZU; HAGA, YOICHI: "Fabrication and electrical characterization of a thin tube-shaped neural electrodes with endoscopic observation function", INSTITUTE OF ELECTRICAL ENGINEERS OF JAPAN MATERIALS MICROMACHINE AND SENSOR SYSTEM STUDY GROUP, no. MSS-18-9, 12 July 2018 (2018-07-12), JP, pages 11 - 14, XP009541019

## Description

### TECHNICAL FIELD

The present disclosure relates to a tube assembly for a living organism and a measurement apparatus for a living organism.

### BACKGROUND OF INVENTION

A known technique is described in, for example, Patent Literature 1. Document WO 2017/170662 A1 describes an optical imaging apparatus comprising a sheath and a pipe for holding the endoscope probe, the pipe provided on the sheath. A lens is covered with the sheath and disposed at its distal end. The sheath may be made of a ceramic material.

### CITATION LIST

### PATENT LITERATURE

Patent Literature 1: WO 2011/132756
Patent Literature 2: WO 2012/017950
Patent Literature 3: Japanese Unexamined Patent Application Publication (Translation of PCT Application) No. 2010-540202
Patent Literature 4: Japanese Patent No. 5224482

### SUMMARY

In an embodiment according to the present disclosure, a tube assembly for a living organism includes a tube and a first ferrule. The tube is partially placeable into a living organism. The tube includes a first end, a second end, a through-hole, and a lens. The through-hole extends in a first direction from the second end to the first end. The first ferrule covers an outer periphery of the tube in the first direction. The lens is located in a portion of the through-hole including at least the first end. The tube and the first ferrule contain a ceramic material.

### BRIEF DESCRIPTION OF THE DRAWINGS

The objects, features, and advantages of the present disclosure will become more apparent from the following detailed description and the drawings.
FIG. 1 is a plan view of a tube assembly for a living organism according to an embodiment of the present disclosure as viewed in y-direction.
FIG. 2 is an exploded plan view of the tube assembly for a living organism according to the embodiment of the present disclosure as viewed in y-direction.
FIG. 3 is a perspective view of a tube in the tube assembly for a living organism illustrated in FIG. 2.
FIG. 4 is a sectional view of the tube illustrated in FIG. 3 taken in x-direction.
FIG. 5 is an enlarged view of part V of the tube illustrated in FIG. 4.
FIG. 6 is a plan view of a tube assembly for a living organism according to another embodiment as viewed in y-direction.
FIG. 7 is a plan view of a first ferrule in the tube assembly for a living organism illustrated in FIG. 2 as viewed in y-direction.
FIG. 8 is a sectional view of the first ferrule illustrated in FIG. 7 taken in x-direction.
FIG. 9 is a plan view of a second ferrule in the tube assembly for a living organism illustrated in FIG. 2 as viewed in y-direction.
FIG. 10 is a sectional view of the second ferrule illustrated in FIG. 9 taken in x-direction.
FIG. 11 is a perspective view of a sleeve in the tube assembly for a living organism illustrated in FIG. 2.
FIG. 12 is a sectional view of the sleeve illustrated in FIG. 11 taken in x-direction.
FIG. 13 is a plan view of a measurement apparatus for a living organism according to an embodiment of the present disclosure as viewed in y-direction.

### DESCRIPTION OF EMBODIMENTS

A tube assembly for a living organism and a measurement apparatus for a living organism are used to, for example, record information on neural activity over a predetermined period from the brains of small animals including rodents, such as mice or rats, and marmosets. The structure that forms the basis of a tube assembly for a living organism and a measurement apparatus for a living organism according to one or more embodiments of the present disclosure includes, for example, an elongated object such as a wire electrode implanted and fixed in the brain of a small animal. Non-claimed example methods used to fix such a wire electrode in a living organism include directly implanting and fixing a linear wire electrode of, for example, relatively rigid stainless steel or stainless alloy in the living organism, or using guide members, for example, screws, to fasten the wire electrode.

A tube assembly 1 for a living organism and a measurement apparatus 100 for a living organism according to one or more embodiments of the present disclosure will now be described with reference to the drawings. The orthogonal xyz coordinate system may be used herein for ease of explanation.

### Structure of Tube Assembly 1 for Living Organism

In one or more embodiments of the present disclosure, the tube assembly 1 for a living organism (hereafter referred to as the tube assembly 1) is used to obtain information on, for example, neural activity from the brain of a living organism 60, for example, a small experimental animal such as a rodent or a marmoset, or an experimental primate such as a monkey or a chimpanzee. The tube assembly 1 is also used to obtain information on cell activity in, for example, an organ or information on the blood flow in a blood vessel. The tube assembly 1 is also used for medical care, for example, treating people or animals.

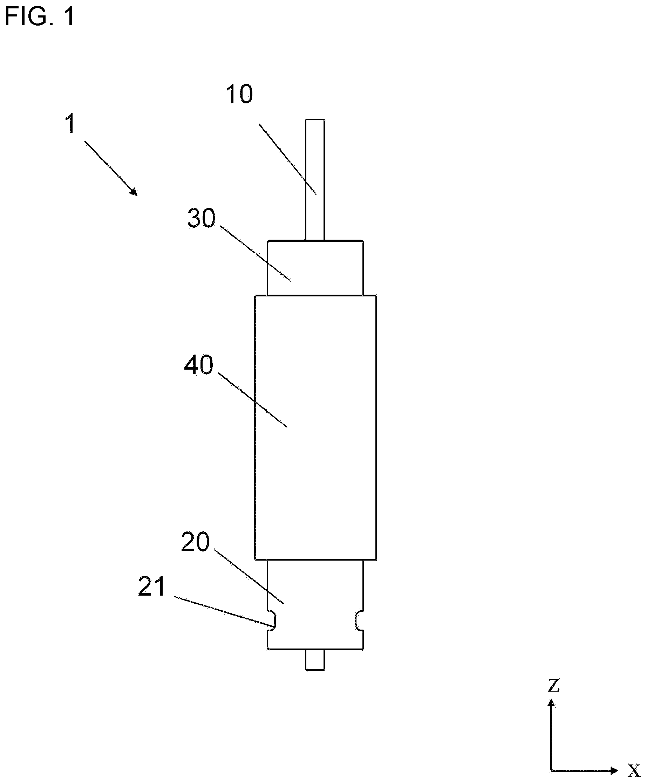

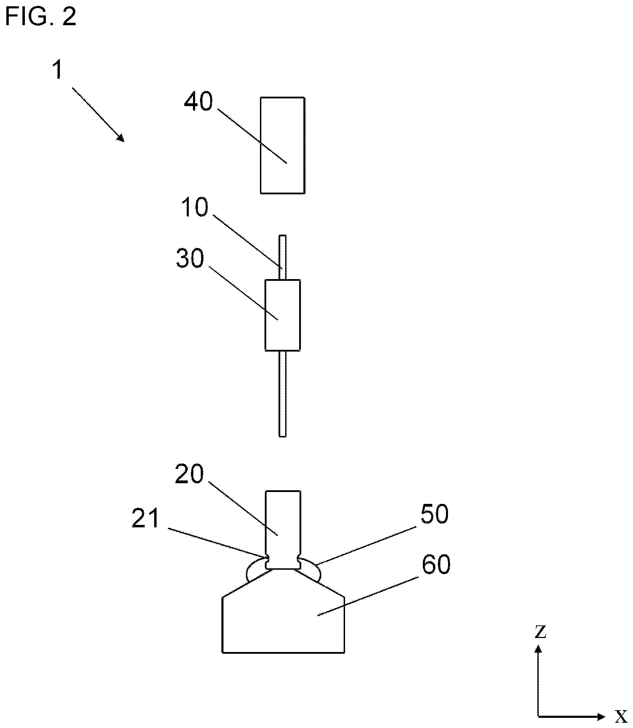

In one or more embodiments of the present disclosure, the tube assembly 1 includes a tube 10 and a first ferrule 20. As illustrated in FIG. 1, the tube assembly 1 may include the tube 10, the first ferrule 20, a second ferrule 30, and a sleeve 40. As illustrated in FIG. 2, the tube 10 is partially placed in the living organism 60. The tube 10 includes a first end 11, a second end 12, a through-hole 13, and a lens 14. The through-hole 13 extends in a first direction from the second end 12 to the first end 11. The lens 14 is located in a portion of the through-hole 13 including at least the first end 11. The first ferrule 20 covers the outer periphery of the tube 10 in the first direction. The first direction herein is from the second end 12 to the first end 11. The through-hole 13 in the tube 10 receives an imaging fiber 70 placed in the first direction.

The tube assembly 1 including, as illustrated in FIG. 1, the tube 10, the first ferrule 20, the second ferrule 30, and the sleeve 40 is connected to the living organism 60 as described below. First, the tube 10 including the first ferrule 20 on its outer periphery is fixed with the first end 11 placed through, for example, an organ, the scalp, or the skull of the living organism 60. The living organism 60 that is a small experimental animal or an experimental primate may be kept with the first ferrule 20 and the tube 10 fixed to the living organism 60. To conduct an experiment for obtaining information on, for example, neural activity or to perform treatment, the imaging fiber 70 connected to the through-hole 31 in the second ferrule 30, or in other words, located in the through-hole 31, is placed in the through-hole 13 in the tube 10. The outer peripheries of the first ferrule 20 and the second ferrule 30 are fastened by the sleeve 40. Light through the lens 14 in the tube 10 travels through the imaging fiber 70 connected to an imaging system 80 described later. The imaging system 80 processes the optical information and measures, for example, neural activity in the living organism 60. With the first ferrule 20 and the tube 10 constantly fixed to the living organism 60, the living organism 60 does not undergo repeated insertion and removal of an object for every experiment. The tube assembly 1 is thus less invasive to the living organism 60. This structure also allows measurement of the same part of the living organism 60 and increases the reliability of the measurement results. To fix the tube assembly 1 to the living organism 60, an operator uses tweezers to hold the tube assembly 1 and attaches the tube assembly 1 to a target site to be measured, for example, an organ, the scalp, or the skull.

As illustrated in FIG. 1, the first end 11 of the tube 10 may protrude from the first ferrule 20. This facilitates placement of the tube 10 in the living organism 60. The tube assembly 1 is thus easily operable.

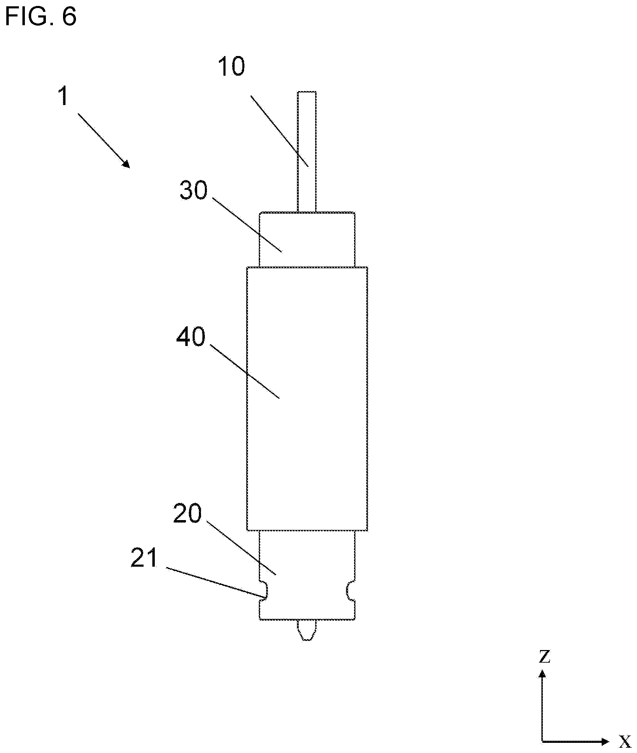

As illustrated in FIG. 6, the tube 10 has an outer diameter that may be gradually smaller toward the first end 11. This allows the tube 10 to be placed in the living organism 60 more easily. Additionally, the living organism 60 suffers less damage during the insertion. The tube assembly 1 is thus less invasive to the living organism 60.

The tube 10 is made of a ceramic material. This reduces allergic reactions, such as metal allergy, in the living organism 60 compared with when, for example, the tube 10 is made of metal. The tube assembly 1 is thus less invasive to the living organism 60. The tube 10 made of a ceramic material also facilitates measurement of the living organism 60 with, for example, magnetic resonance imaging (MRI).

Examples of the ceramic material used for the tube 10 include alumina (Al₂O₃), zirconia (ZrO₂), aluminum nitride (AlN), silicon carbide (SiC), silicon nitride (Si₃N₄), forsterite (2MgO·SiO₂), sialon (SiAlON), barium titanate (BaTiO₃), lead zirconate titanate (PZT), ferrite, and mullite. For the tube 10 made of a zirconia ceramic material, fine particles of zirconia allow the tube 10 to have more accurate dimensions. In some embodiments, the tube 10 made of a zirconia ceramic material may include an additive. The additive may be, for example, a stabilizer such as yttria (Y₂O₃). The tube 10 may thus be tougher.

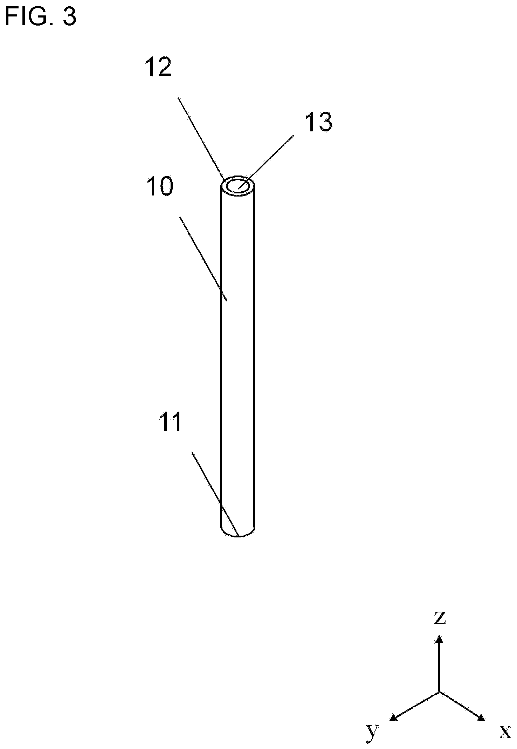

As illustrated in FIG. 3, the tube 10 may be, for example, hollow cylindrical. For the tube 10 that is hollow cylindrical, the first end 11 and the second end 12 may each have a diameter of, for example, 0.5 to 3.0 mm. The size in z-direction may be 5 to 30 mm. The through-hole 13 may have a diameter of 0.3 to 2.0 mm.

The lens 14 is located in the through-hole 13 in the tube 10 and may have the same shape as the through-hole 13 in the tube 10. More specifically, the lens 14 may be solid cylindrical. For the lens 14 that is solid cylindrical, its end in the positive z-direction may have a diameter of, for example, 0.3 to 2.0 mm, and its end in the negative z-direction may have a diameter of, for example, 0.3 to 2.0 mm. The size in z-direction may be 0.3 to 3.0 mm.

The lens 14 may be, for example, a rod lens or a GRIN lens.

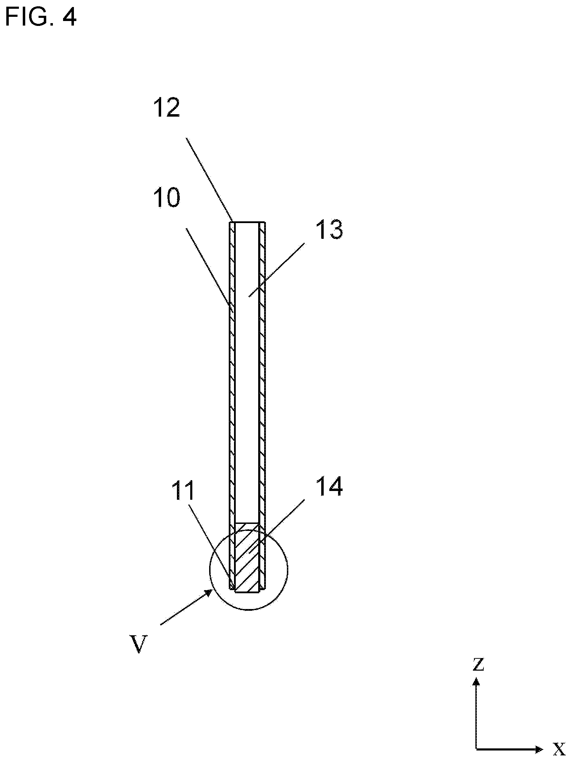

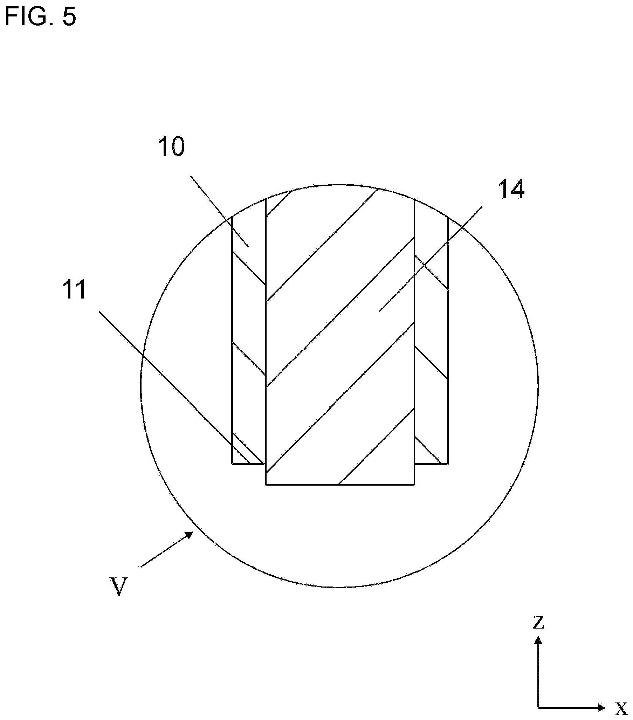

As illustrated in FIG. 5, which is an enlarged view of FIG. 4, the lens 14 may protrude from the first end 11. This structure allows the lens 14 to come in contact with a site to be measured in the living organism 60 and improves the accuracy of data collection. The tube assembly 1 including the lens 14 has high measurement accuracy.

For the tube assembly 1 including the first ferrule 20, the first ferrule 20 functions as a stopper when the first end 11 is placed through, for example, the scalp and the skull of the head of a small experimental animal. With the first ferrule 20 functioning as a stopper, the tube assembly 1 is more easily fixed and is less likely to be placed deeper in the living organism 60 than an appropriate depth when the tube assembly 1 is connected to the living organism 60. The tube assembly 1 is thus more stably connected and is less invasive to the living organism 60. The first ferrule 20 fastens the outer periphery of the tube 10, and the through-hole 13 in the tube 10 and the through-hole 22 in the first ferrule 20 are coaxial.

The first ferrule 20 may include a recess 21 open on its outer periphery. The recess 21 may receive a retainer 50 described later. The recess 21 may have a rough surface. A rough surface refers to a surface having a greater surface roughness than other portions. The surface roughness may be measured and calculated by a stylus method as a contact method, or by a light interferometry method, a focus-variation image composition method, or a confocal method as a non-contact method. The measurement method may be selected as appropriate based on, for example, the size and the shape of a target object.

As illustrated in FIG. 2, the tube assembly 1 may include the retainer 50 that extends from the outer periphery of the first ferrule 20 to the living organism 60 and is connected to the living organism 60. More specifically, the retainer 50 fastens the living organism 60 and the first ferrule 20 together. This structure allows the tube assembly 1 to be firmly fixed to the living organism 60.

When the first ferrule 20 includes the recess 21, the retainer 50 may be located at the recess 21. This structure increases the area of contact between the retainer 50 and the first ferrule 20 and improves the strength of connection between the living organism 60 and the first ferrule. The recess 21 including a rough surface further improves the strength of connection.

The first ferrule 20 is made of a ceramic material. This reduces allergic reactions, such as metal allergy, in the living organism 60. The tube assembly 1 is thus less invasive to the living organism 60. The first ferrule 20 made of a ceramic material also facilitates measurement of the living organism 60 with, for example, MRI.

Examples of the ceramic material used for the first ferrule 20 include Al₂O₃, ZrO₂, AlN, SiC, Si₃N₄, 2MgO·SiO₂, SiAlON, BaTiO₃, PZT, ferrite, and mullite. The first ferrule 20 made of a zirconia ceramic material can have more accurate dimensions. In some embodiments, the first ferrule 20 made of a zirconia ceramic material may include an additive. The additive may be, for example, a stabilizer such as Y₂O₃. The first ferrule 20 may thus be tougher.

The entire outer peripheral surface of the first ferrule 20 may be rough. The first ferrule 20 may thus be held by tweezers more firmly and be less likely to be out of position in connecting to the living organism 60. The tube assembly 1 thus has high connection reliability.

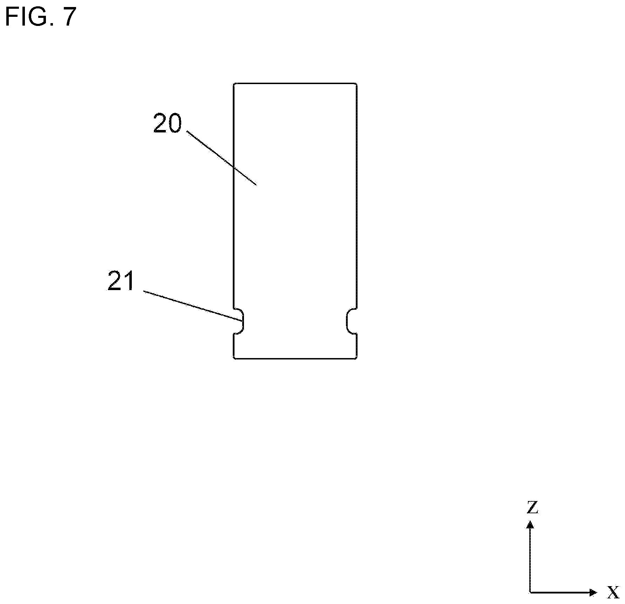

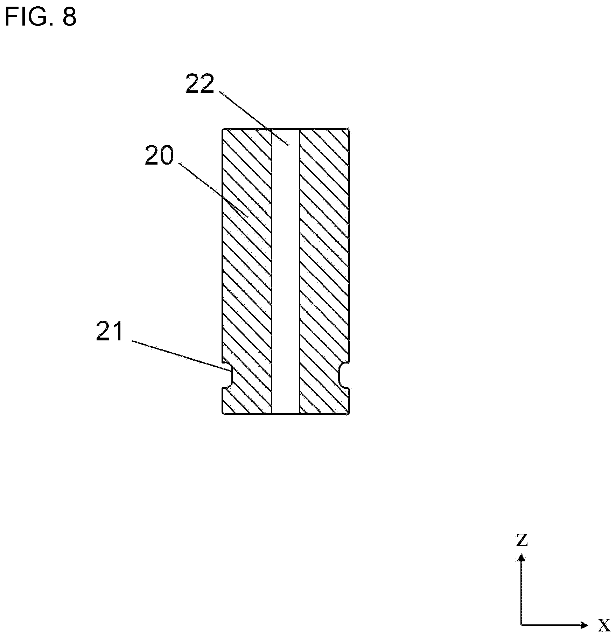

The first ferrule 20 illustrated in FIGs. 7 and 8 may be, for example, hollow cylindrical. For the first ferrule 20 that is hollow cylindrical, its end in the positive z-direction may have a diameter of, for example, 1.0 to 5.0 mm, and its end in the negative z-direction may have a diameter of, for example, 1.0 to 5.0 mm. The size in z-direction may be 5 to 30 mm. The through-hole 22 may have a diameter of 0.5 to 3.0 mm.

The retainer 50 may be made of a resin, such as an epoxy resin. The retainer 50 made of a resin may dry faster and thus be less damaging to the living organism 60. The tube assembly 1 is thus less invasive to the living organism 60.

The tube assembly 1 may include the second ferrule 30 that fastens the outer periphery of the imaging fiber 70. This structure allows the imaging fiber 70 to be accurately connected to the first ferrule 20, as well as allowing the imaging fiber 70 to be less likely to break due to, for example, an impact. The imaging fiber 70 is placed in the through-hole 13 in the tube 10 to be fastened by the tube 10. In this structure, the through-hole 31 in the second ferrule 30, the through-hole 13 in the tube 10, and the through-hole 22 in the first ferrule 20 are coaxial.

The second ferrule 30 may be made of a ceramic material. This reduces allergic reactions, such as metal allergy, in the living organism 60. The first ferrule 20 made of a ceramic material allows more accurate connection to the second ferrule 30. The second ferrule 30 made of a ceramic material also facilitates measurement of the living organism 60 with, for example, MRI.

Examples of the ceramic material used for the second ferrule 30 include Al₂O₃, ZrO₂, AlN, SiC, Si₃N₄, 2MgO·SiO₂, SiAlON, BaTiO₃, PZT, ferrite, and mullite. The second ferrule 30 made of a zirconia ceramic material can have more accurate dimensions. In some embodiments, the second ferrule 30 made of a zirconia ceramic material may include an additive. The additive may be, for example, a stabilizer such as Y₂O₃. The second ferrule 30 may thus be tougher.

The entire outer peripheral surface of the second ferrule 30 may be rough. The second ferrule 30 may thus be held by tweezers more firmly and be less likely to be out of position in connecting to the living organism 60. The tube assembly 1 thus has high connection reliability.

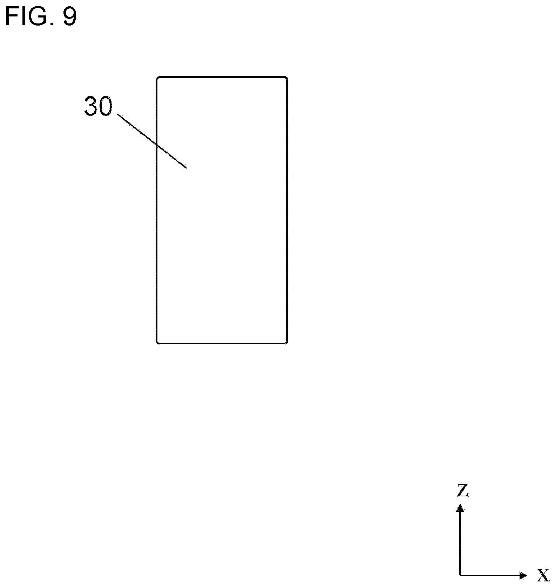

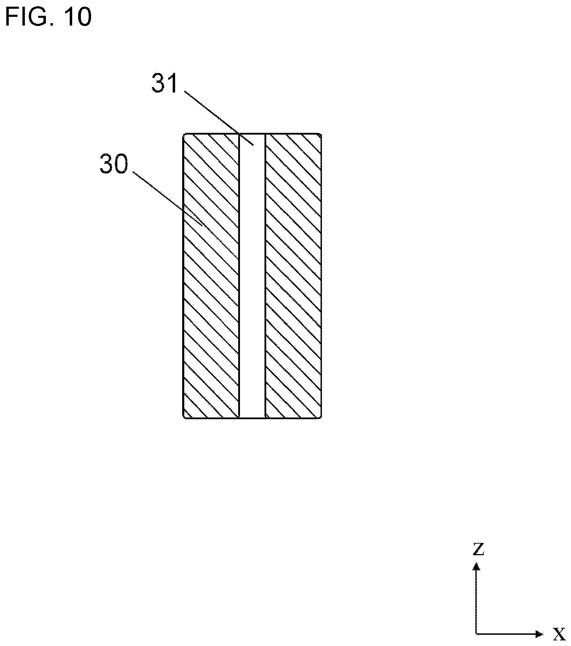

The second ferrule 30 illustrated in FIGs. 9 and 10 may be, for example, hollow cylindrical. For the second ferrule 30 that is hollow cylindrical, its end in the positive z-direction may have a diameter of, for example, 1.0 to 5.0 mm, and its end in the negative z-direction may have a diameter of, for example, 1.0 to 5.0 mm. The size in z-direction may be 5 to 30 mm. The through-hole 31 may have a diameter of 0.5 to 3.0 mm.

The tube assembly 1 may include the sleeve 40 that fastens the outer peripheries of the first ferrule 20 and the second ferrule 30. With the sleeve 40 fastening the outer peripheries of the first ferrule 20 and the second ferrule 30, the through-hole 41 in the sleeve 40, the through-hole 13 in the tube 10, the through-hole 22 in the first ferrule 20, and the through-hole 31 in the second ferrule 30 are coaxial.

The sleeve 40 may be made of a ceramic material. With the first ferrule 20 and the second ferrule 30 made of a ceramic material, the sleeve 40 can be accurately connected to the first ferrule 20 and the second ferrule 30. The sleeve 40 made of a ceramic material also facilitates measurement of the living organism 60 with, for example, MRI.

Examples of the ceramic material used for the sleeve 40 include Al₂O₃, ZrO₂, AlN, SiC, Si₃N₄, 2MgO·SiO₂, SiAlON, BaTiO₃, PZT, ferrite, and mullite. The sleeve 40 made of a zirconia ceramic material may have more accurate dimensions. In some embodiments, the sleeve 40 made of a zirconia ceramic material may include an additive. The additive may be, for example, a stabilizer such as Y₂O₃. The sleeve 40 may thus be tougher.

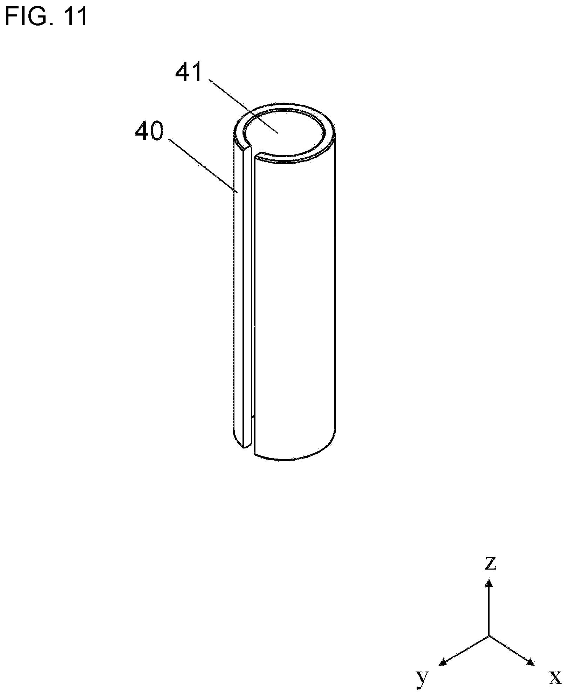

The sleeve 40 may be a split sleeve. In some embodiments, the sleeve 40 may be a precision sleeve. For the sleeve 40 being a split sleeve, the sleeve 40 is elastic and can fasten the first ferrule 20 and the second ferrule 30 firmly. The tube assembly 1 including the sleeve 40 has high connection reliability. A split sleeve includes a slit in z-direction as illustrated in FIG. 11. A precision sleeve does not include a slit in z-direction unlike a split sleeve.

The entire outer peripheral surface of the sleeve 40 may be rough. The sleeve 40 may thus be held by tweezers more firmly and be less likely to be out of position in connecting to the living organism 60. The tube assembly 1 thus has high connection reliability.

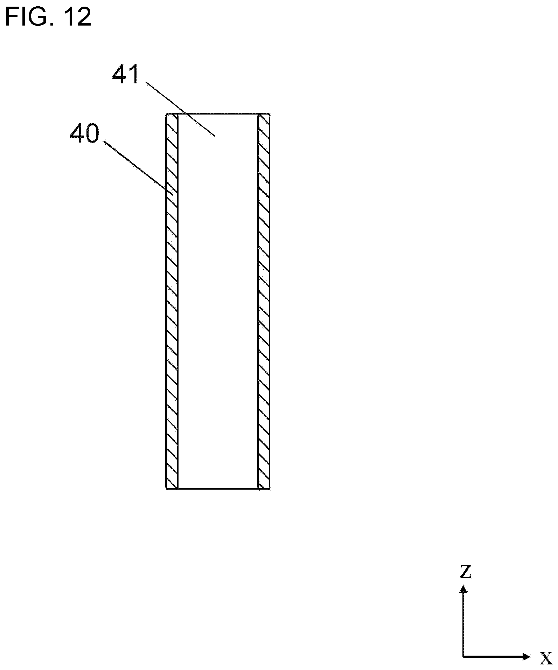

The sleeve 40 illustrated in FIGs. 11 and 12 may be, for example, hollow cylindrical. For the sleeve 40 that is hollow cylindrical, its end in the positive z-direction may have a diameter of, for example, 1.5 to 8.0 mm, and its end in the negative z-direction may have a diameter of, for example, 1.5 to 8.0 mm. The size in z-direction may be 5 to 30 mm. The through-hole 41 may have a diameter of 1.0 to 5.0 mm.

The dimensions of the components in the tube assembly 1 are not limited to the dimensions described above and may be any dimensions appropriate for, for example, the type of a measurement target and a target site to be measured.

The imaging fiber 70 may be an optical fiber of, for example, quartz glass.

When the imaging fiber 70 is placed in the through-hole 13 in the tube 10, the end face of the imaging fiber 70 may be connected to the lens 14.

### Structure of Measurement Apparatus 100 for Living Organism

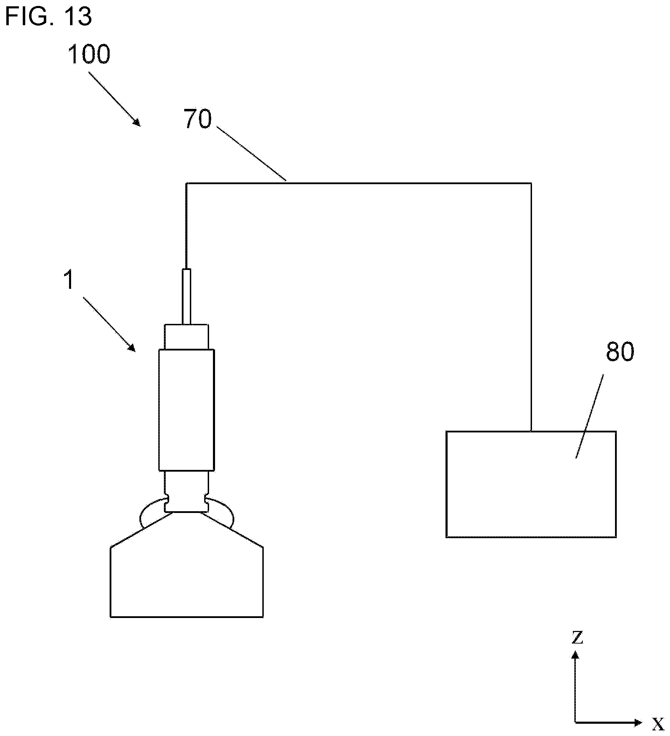

The measurement apparatus 100 for a living organism illustrated in FIG. 13 includes the tube assembly 1 described above and the imaging system 80 connected to the imaging fiber 70.

The imaging system 80 may be, for example, an endoscopic system. Non-claimed method for Manufacturing Tube Assembly 1 for Living Organism

The tube 10 and the first ferrule 20 in the tube assembly 1 may be formed through the processes described below. First, a powder of a ceramic material such as zirconia is kneaded with a thermoplastic binder into a mixture. The mixture is then molded under pressure into a molded body using a mold with a predetermined shape. The molded body is then fired at temperatures of about 1300 to 1600 °C. Through the above processes, the tube 10 and the first ferrule 20 of a ceramic material containing zirconia are formed. The second ferrule 30 and the sleeve 40 may also be formed by the above-described method used to form the tube 10 and the first ferrule 20.

When the first ferrule 20 includes the recess 21 as illustrated in FIG. 7, the mold used to form the first ferrule 20 includes a projection for forming the recess 21. Thus, the recess 21 may be formed. To form a tube 10 including a distal end tapered toward the first end 11, a mold for the tube 10 is processed to be tapered toward its distal end, and the mold is used to form the tube 10.

To create rough surfaces on the outer peripheries of the recess 21 on the first ferrule 20, the first ferrule 20, the second ferrule 30, and the sleeve 40, abrasive blasting of propelling an abrasive material may be used. In some embodiments, targeted portions to be roughened may be immersed in an etching solution to form rough surfaces through chemical erosion. In other embodiments, the rough surfaces may be formed through a surface roughing process, in which a rough surface member made of, for example, a resin is pressed against the portions corresponding to the outer peripheries of the molded bodies to be the first ferrule 20, the second ferrule 30, and the sleeve 40, followed by firing.

The present disclosure may be implemented in the following forms.

In an embodiment according to the present disclosure, a tube assembly for a living organism includes a tube and a first ferrule. The tube is partially placeable into a living organism. The tube includes a first end, a second end, a through-hole, and a lens. The through-hole extends in a first direction from the second end to the first end. The first ferrule covers an outer periphery of the tube in the first direction. The lens is located in a portion of the through-hole including at least the first end. The tube and the first ferrule contain a ceramic material.

In an embodiment of the present disclosure, the tube assembly for a living organism is less invasive to the living organism.

### REFERENCE SIGNS

- 1: tube assembly for living organism
- 10: tube
- 11: first end
- 12: second end
- 13: through-hole
- 14: lens
- 20: first ferrule
- 21: recess
- 22: through-hole
- 30: second ferrule
- 31: through-hole
- 40: sleeve
- 50: retainer
- 60: living organism
- 70: imaging fiber
- 80: imaging system
- 100: measurement apparatus for living organism

## Claims

1. A tube assembly for a living organism, the tube assembly comprising:
a tube partially placeable into a living organism, the tube including a first end, a second end, and a through-hole extending in a first direction from the second end to the first end; and
a first ferrule covering an outer periphery of the tube in the first direction,
wherein the tube includes a lens in a portion of the through-hole including at least the first end, and
the tube and the first ferrule comprise a ceramic material.

2. The tube assembly according to claim 1, wherein
the first end protrudes from the first ferrule.

3. The tube assembly according to claim 1 or claim 2, wherein
the tube has an outer diameter gradually smaller toward the first end.

4. The tube assembly according to any one of claims 1 to 3, wherein
the lens protrudes from the first end.

5. The tube assembly according to any one of claims 1 to 4, further comprising:
a retainer extending from an outer periphery of the first ferrule and to be connected to the living organism.

6. The tube assembly according to claim 5, wherein
the first ferrule includes a recess being open on the outer periphery of the first ferrule, and
the retainer is located at the recess.

7. The tube assembly according to claim 5 or claim 6, wherein
the retainer comprises a resin.

8. The tube assembly according to any one of claims 1 to 7, wherein
the tube comprises a zirconia ceramic material.

9. The tube assembly according to any one of claims 1 to 8, further comprising:
an imaging fiber connectable to the lens located in the through-hole.

10. The tube assembly according to claim 9, further comprising:
a second ferrule configured to fasten an outer periphery of the imaging fiber, the second ferrule comprising a ceramic material; and
a sleeve configured to fasten outer peripheries of the first ferrule and the second ferrule, the sleeve comprising a ceramic material.

11. The tube assembly according to claim 10, wherein
the sleeve is a split sleeve.

12. A measurement apparatus for a living organism, the measurement apparatus comprising:
the tube assembly according to any one of claims 9 to 11; and
an imaging system connectable to the imaging fiber.

## Patentansprüche

1. Röhrchenbaugruppe für einen lebenden Organismus, wobei die Röhrchenbaugruppe aufweist:
ein Röhrchen, das teilweise in einem lebenden Organismus platzierbar ist, wobei das Röhrchen ein erstes Ende, ein zweites Ende und ein Durchgangsloch aufweist, das sich in einer ersten Richtung vom zweiten Ende zum ersten Ende erstreckt, und
eine erste Hülse, die einen Außenumfang des Röhrchens in der ersten Richtung bedeckt,
wobei das Röhrchen eine Linse in einem Abschnitt des Durchgangslochs aufweist, der mindestens das erste Ende aufweist, und
das Röhrchen und die erste Hülse ein Keramikmaterial aufweisen.

2. Röhrchenbaugruppe gemäß Anspruch 1, wobei
das erste Ende von der ersten Hülse hervorsteht.

3. Röhrchenbaugruppe gemäß Anspruch 1 oder Anspruch 2, wobei das Röhrchen einen Außendurchmesser hat, der in Richtung zum ersten Ende allmählich kleiner wird.

4. Röhrchenbaugruppe gemäß irgendeinem der Ansprüche 1 bis 3, wobei
die Linse von dem ersten Ende hervorsteht.

5. Röhrchenbaugruppe gemäß irgendeinem der Ansprüche 1 bis 4, ferner aufweisend:
eine Haltevorrichtung, die sich von einem Außenumfang der ersten Hülse erstreckt und mit dem lebenden Organismus zu verbinden ist.

6. Röhrchenbaugruppe gemäß Anspruch 5, wobei
die erste Hülse eine am Außenumfang der ersten Hülse offene Aussparung aufweist und die Haltevorrichtung an der Aussparung angeordnet ist.

7. Röhrchenbaugruppe gemäß Anspruch 5 oder Anspruch 6, wobei
die Haltevorrichtung ein Harz aufweist.

8. Röhrchenbaugruppe gemäß irgendeinem der Ansprüche 1 bis 7, wobei
das Röhrchen ein Zirkonoxid-Keramikmaterial aufweist.

9. Röhrchenbaugruppe gemäß irgendeinem der Ansprüche 1 bis 8, ferner aufweisend:
eine Bildgebungsfaser, die mit der in dem Durchgangsloch angeordneten Linse verbindbar ist.

10. Röhrchenbaugruppe gemäß Anspruch 9, ferner aufweisend:
eine zweite Hülse, die konfiguriert ist, um einen Außenumfang der Bildgebungsfaser zu befestigen, wobei die zweite Hülse ein Keramikmaterial aufweist, und
eine Manschette, die konfiguriert ist, um Außenumfänge der ersten Hülse und der zweiten Hülse zu befestigen, wobei die Manschette ein Keramikmaterial aufweist.

11. Röhrchenbaugruppe gemäß Anspruch 10, wobei
die Manschette eine geteilte Manschette ist.

12. Messvorrichtung für einen lebenden Organismus, wobei die Messvorrichtung aufweist:
die Röhrchenbaugruppe gemäß irgendeinem der Ansprüche 9 bis 11 und
ein Bildgebungssystem, das mit der Bildgebungsfaser verbindbar ist.

## Revendications

1. Ensemble de tube pour un organisme vivant, l'ensemble de tube comprenant :
un tube pouvant être partiellement placé dans un organisme vivant, le tube comprenant une première extrémité, une deuxième extrémité et un trou traversant s'étendant dans une première direction depuis la deuxième extrémité vers la première extrémité ; et
une première virole recouvrant une périphérie extérieure du tube dans la première direction,
dans lequel le tube comprend une lentille dans une partie du trou traversant comprenant au moins la première extrémité, et
le tube et la première virole comprennent un matériau céramique.

2. Ensemble de tube selon la revendication 1, dans lequel
la première extrémité fait saillie de la première virole.

3. Ensemble de tube selon la revendication 1 ou la revendication 2, dans lequel le tube a un diamètre extérieur progressivement plus petit vers la première extrémité.

4. Ensemble de tube selon l'une quelconque des revendications 1 à 3, dans lequel
la lentille fait saillie de la première extrémité.

5. Ensemble de tube selon l'une quelconque des revendications 1 à 4, comprenant en outre :
un dispositif de retenue s'étendant depuis une périphérie extérieure de la première virole et destiné à être connecté à l'organisme vivant.

6. Ensemble de tube selon la revendication 5, dans lequel
la première virole comprend un évidement ouvert sur la périphérie extérieure de la première virole, et
le dispositif de retenue est situé au niveau de l'évidement.

7. Ensemble de tube selon la revendication 5 ou la revendication 6, dans lequel
le dispositif de retenue comprend une résine.

8. Ensemble de tube selon l'une quelconque des revendications 1 à 7, dans lequel
le tube comprend un matériau céramique de zircone.

9. Ensemble de tube selon l'une quelconque des revendications 1 à 8, comprenant en outre :
une fibre d'imagerie pouvant être connectée à la lentille située dans le trou traversant.

10. Ensemble de tube selon la revendication 9, comprenant en outre :
une deuxième virole configurée pour fixer une périphérie extérieure de la fibre d'imagerie, la deuxième virole comprenant un matériau céramique ; et
un manchon configuré pour fixer les périphéries extérieures de la première virole et de la deuxième virole, le manchon comprenant un matériau céramique.

11. Ensemble de tube selon la revendication 10, dans lequel
le manchon est un manchon fendu.

12. Appareil de mesure pour un organisme vivant, l'appareil de mesure comprenant :
l'ensemble de tube selon l'une quelconque des revendications 9 à 11 ; et
un système d'imagerie pouvant être connecté à la fibre d'imagerie.
